# EUROPEAN PATENT SPECIFICATION

(11) **EP 1 957 159 B1**
(45) Date of publication and mention of the grant of the patent: **09.09.2015**
(21) Application number: 05813446.1
(22) Date of filing: 30.11.2005
(51) Int. Cl.: A61N 1/368, A61N 1/362, A61N 1/365

(54) **IMPLANTABLE CARDIAC STIMULATOR FOR MONITORING THE HEART CYCLE IN A HUMAN HEART**
IMPLANTIERBARER HERZSTIMULATOR ZUR ÜBERWACHUNG DES HERZZYKLUS IM MENSCHLICHEN HERZ
STIMULATEUR CARDIAQUE IMPLANTABLE POUR SURVEILLER LE CYCLE CARDIAQUE DANS UN COEUR HUMAIN

(43) Date of publication of application: 20.08.2008
(73) Proprietor: St. Jude Medical AB, 175 84 Järfälla (SE)
(72) Inventor: JÄRVERUD, Karin, 169 71 Solna (SE)
(74) Representative: Sjölander, Henrik
(86) International application number: PCT/SE2005/001808
(87) International publication number: WO 2007/064261

(56) References cited:
- US-A- 4 917 115
- US-A1- 2002 151 938
- US-A1- 2003 100 925
- US-A1- 2003 105 496
- US-A1- 2004 019 365
- US-A1- 2004 172 078
- US-A1- 2005 027 320
- US-A1- 2005 209 649
- US-A1- 2006 161 211
- US-A1- 2006 178 586

## Description

### Technical field

The present invention generally relates to the field of implantable heart stimulation devices, such as pacemakers, implantable cardioverter-defibrillators (ICD), and similar cardiac stimulation devices that also are capable of monitoring and detecting electrical activities and events within the heart. More specifically, the present invention relates to a device for monitoring the heart cycle of a human heart such that coronary flow may be maintained at a desired level, a cardiac stimulator comprising such a device, and a method of monitoring the heart cycle of a human heart.

### Background art

Implantable heart stimulators that provide stimulation pulses to selected locations in the heart, e.g. selected chambers, have been developed for the treatment of cardiac diseases and dysfunctions. Heart stimulators have also been developed that affect the manner and degree to which the heart chambers contract during a cardiac cycle in order to promote the efficient pumping of blood. The heart will pump more effectively when a coordinated contraction of both atria and both ventricles can be provided. In a healthy heart, the coordinated contraction is provided through conduction pathways in both the atria and the ventricles that enable a very rapid conduction of electrical signals to contractile tissue throughout the myocardium to effectuate the atrial and ventricular contractions. If these conduction pathways do not function properly, a slight or severe delay in the propagation of electrical pulses may arise, causing asynchronous contraction of the ventricles which would greatly diminish the pumping efficiency of the heart. Patients who exhibit pathology of these conduction pathways, such as patients with bundle branch blocks, etc., can thus suffer compromised pumping performance.

Various prior art procedures have been developed for addressing these and other disorders. For instance, cardiac resynchronization therapy (CRT) can be used for effectuating synchronous atrial and/or ventricular contractions. Furthermore, cardiac stimulators may be provided that deliver stimulation pulses at several locations in the heart simultaneously, such as biventricular stimulators. The stimulation pulses could also be delivered to different locations with a selected delay in an attempt to optimize the hemodynamic performance, e.g. maximize cardiac output, in relation to the specific cardiac dysfunction present at the time of implant. During follow-up, a physician may alter the delay settings in adaptation to altered cardiac status. However, none of the prior art procedures addresses the issue of maintaining the coronary flow for providing oxygen-rich blood to the myocardium at a desired level.

US 2003/0100925 discloses an implantable device for measuring mechanical heart function using a magnetic field sensor. The output signal of the magnetic field sensor has a signal magnitude proportional to the magnetic field strength of one or more magnets, which in turn is indicative of changing cardiac dimensions.

US 2004/0019365 discloses automatic adjustment of stimulation parameters in response to contraction intervals measured by accelerometers and compared to optimal contraction intervals.

### Summary of the invention

An object of the present invention is to address the problem of maintaining coronary flow at a desired level.

This and other objects are achieved by a device as claimed in the independent claim and a cardiac stimulator comprising such a device. Further embodiments are defined in the dependent claims.

According to one aspect of the present invention, there is provided a device for monitoring the heart cycle of a human heart such that coronary flow may be maintained at a desired level, the device being connected to a first sensor adapted to be positioned at a first location of the heart and arranged for sensing cardiac wall movements at said first location, and to a second sensor adapted to be positioned at a second location of the heart and arranged for sensing cardiac wall movements at said second location. The device comprises processing circuitry arranged for receiving output signals from said first and second sensors, said output signals being indicative of myocardial relaxation at said first and second locations, determining the time of myocardial relaxation at said first and second locations, and providing a diastolic synchronization signal indicative of the synchrony or synchronicity in the time of myocardial relaxation between said first and said second location.

According to another aspect of the present invention, there is provided an implantable cardiac stimulator for delivering stimulation pulses to a human heart. The cardiac stimulator comprises a housing, a pulse generator enclosed in said housing for generating said stimulation pulses, control circuitry for controlling the delivery of said stimulation pulses to the heart, and a device for monitoring the heart cycle of a human heart as described above, the stimulator being connectable to a lead arrangement for conducting said stimulation pulses to the heart and sensed electrical signals from the heart to the control circuitry. It is to be noted that the term "implantable cardiac stimulator" is intended to encompass any implantable device arranged for providing electrical stimuli for controlling the operation of a human heart, such as an ICD or a pacemaker, e.g. of biventricular, dual-chamber, AV-sequential, or any other type known in the art.

Thus, the present invention is based on the advantageous idea of monitoring the heart cycle for determining the synchronization of myocardial relaxation during the diastolic phase of the heart cycle. Thereby, conditions that are necessary for maintaining coronary flow at a desired level can be monitored, which enables the detection of the demand for suitable corrective action when the conditions change or deteriorate.

All coronary blood supply, or cardiac perfusion, occurs during the diastolic phase of the heart cycle, i.e. when the myocardium relaxes between contractions. At the onset of the systolic phase, the myocardial tissue is contracted, thereby also contracting the coronary arteries and arterioles such that coronary flow virtually comes to a stop during systole. When the myocardial tissue relaxes and dilates, the arteries and arterioles also become dilated and the pressure gradient built up during the systolic phase forces the flow of blood through the coronary arteries and veins. Thus, the diastolic phase should be sufficiently long for providing sufficient time for coronary flow to occur.

It has been found that disturbances in the diastolic phase may have an adverse effect on the cardiac perfusion, i.e. such that the coronary flow is decreased. Thereby, the myocardium may not get the amount of oxygen that is required for a sufficient or desired cardiac performance, resulting in an ischemic situation. Such disturbances can be due to asynchronous systole, and post-systolic contractions (PSC). All of these may result in localized contractions of the myocardium during the diastolic phase, that will disturb the coronary flow locally and may also disturb or prevent a simultaneous onset of the diastolic phase.

As understood from the above, in cardiac stimulation therapy, the stimulator settings should be adapted such that a synchronous diastole with an adequately long duration is achieved. Thus, the aim at the time of implant would be to localize a region with the occurrence of possible PSC that would disturb the synchronous onset or duration of diastole, and then to stimulate the heart using a stimulation therapy pattern such that the occurrences of PSC is removed or decreased. This can be performed by adjusting the timing parameters for the delivery of the stimulation pulses.

However, even though ventricular and atrial diastolic synchrony may be present at the time of implant, possibly supported by suitable cardiac stimulation therapy, this may not necessarily be the case at a later stage. For instance, during progression of cardiac therapy after implantation of a cardiac stimulator, the cardiac tissue may adapt itself to the new conditions. Then, the function of hibernating myocardial tissue may be at least partially restored, and the overall cardiac function may become different from that at the time of implant.

In other words, diastolic synchrony from the time of implant may turn into diastolic asynchrony at a later stage, possibly supported or induced by stimulation therapy, as a result of a local improvement in the local function of myocardial tissue. For instance, the functions of myocardial portions or regions that at the time of implant were affected by slow conduction or post-systolic contractions (PSC), could at a later stage have improved their behavior such that there is no longer any slow conduction or PSC, or the PSC patterns have changed. Thus, even though there is an improvement in the behavior of myocardial tissue through the remodulation or recovery of the heart during progression of cardiac therapy, a new cardiac situation has arisen by this improvement and an improvement of the changed overall function of the heart may be desired for adaptation to the new situation.

According to the invention, the heart cycle is monitored by sensing the heart wall movements during the heart cycle at a plurality of locations. The heart wall movements are sensed using at least two sensors that are attached to the heart wall and provide output signals indicative of the heart wall movements, in order for distinguishing between contractions and relaxations of the muscular heart wall tissue, i.e. the myocardium. The output signals from each sensor are provided through cardiac leads to processing circuitry provided in an implantable heart stimulator and used for determining onset and cessation of myocardial relaxation at the location of the sensor.

The processing circuitry receives the output signals provided by the respective sensors and determines any differences in time of myocardial relaxation for the myocardial tissue at the respective sensor location. If there are no differences, an indication of diastolic synchrony may be provided, which means that a satisfactory coronary flow is enabled.

The term "time of myocardial relaxation" may refer to the point in time of onset of myocardial relaxation, which indicates the onset of the diastolic phase. If there is a time difference between the onsets of myocardial relaxation at one sensor as compared to at the other sensor(s), the coronary flow may be greatly impaired during the time of asynchrony, i.e. when a portion of the myocardium is relaxed and another portion is contracted. Thereby, the time available between ventricular systole for supplying oxygen to the myocardial tissue is compromised, possibly resulting in impaired cardiac performance.

Furthermore, the term "time of myocardial relaxation" may additionally or alternatively refer to the time duration of myocardial relaxation, i.e. the portion of the diastolic phase that is not interrupted by any myocardial contraction, such as a regular systolic contraction or a PSC. Thus, not only the onset of myocardial relaxation is determined, but also the time duration during which the myocardium is in the relaxed state. Thereby, the time duration of simultaneous relaxation, i.e. diastole, for the myocardial locations at which the sensors are positioned may be determined, which indicates the time duration when the blood may flow through and oxygenate the myocardium. Thus, this time duration should be as large as possible for enabling optimal oxygenation of the myocardium during the heart cycle.

Since it is the diastolic phase that is of interest for the calculation of the synchronization index, an intracardiac electrogram, IEGM, could be used for providing an indication related to when the output signals of the sensors may be used for determining onset and end of the diastolic phase. Then, the processing circuitry could be arranged to only receive sensor output signals provided during the diastolic phase. Thereby, there will be no risk of misinterpreting an asynchrony that may be present in the systolic phase as an asynchrony in the diastolic phase.

Moreover, the processing circuitry calculates a synchronization signal or index, related to the diastolic phase, on the basis of the output signals from the respective sensors. These calculations could be performed in a plurality of different manners, as understood by the person skilled in the art. For instance, the synchronization index or signal could be the actual difference time for the sensed onset of myocardial relaxation between the different sensors. In another example, the index could be related to the duration of simultaneous myocardial relaxation, for instance as a percentage of a measured or calculated optimal time duration.

In a further example, the difference between the sensor output signals could be calculated, for the diastolic phase, for instance by simply subtracting one output signal from another. The resulting difference signal could then be used as said synchronization signal per se, or statistical calculations could be applied to the difference signal to arrive at a suitable value indicative of the synchronization. If more than two sensors are used, a plurality of difference signals could be provided, for selected sensor combinations or for all combinations. The plural difference signals could then simply be aggregated to obtain a synchronization signal that would take into account all sensors, or be subject to suitable statistical calculations to arrive at a synchronization index.

In yet further examples, the synchronization index or signal could be obtained through plotting of the sensor output signals in x-y plots and detecting patterns between the plots, for instance by cross-correlation, neural network signal processing, or loop discrimination. Such loop discrimination is disclosed in U.S. Patents Nos. 5,427,112 and 5,556,419, which are incorporate herein by reference.

However, the present invention is not intended to be restricted to the examples of methods for calculating a synchronization index or signal presented herein. On the contrary, any suitable method for calculating a synchronization signal or index from the output signals of the sensors is contemplated within the scope of the present invention.

According to embodiments of the present invention, the processing circuitry is arranged for comparing the obtained synchronization index or signal with a threshold value or signal. Then, the threshold value would be an indicator whether the diastolic synchronization lies within an acceptable range or not. In other words, as long as the synchronization index is within a selected range, as defined by one or more threshold values, a desired level of coronary flow is considered to be enabled. However, should the synchronization index fall outside the intended range, an indication of diastolic asynchrony, or insufficient diastolic synchrony, may be provided.

Such an indication could in exemplifying embodiments of the invention be used for triggering a change in the stimulation therapy for the patient. Such a change could for example refer to an adjustment in the VV-interval, e.g. for a biventricular heart stimulator; a change in the AV-interval, e.g. for a dual chamber or an AV-sequential heart stimulator; or combinations thereof. Thereby, the diastolic synchrony can be monitored during remodulation of the patient's heart, and the pacing therapy can be adjusted in adaptation to the remodulation such that the coronary flow may be maintained at a desired level.

The changes in the timing parameters of the stimulation pulse delivery can, according to exemplifying embodiments, be performed in order to avoid or eliminate an asynchrony in the onset of the myocardial relaxation, i.e. an onset of the diastolic phase. Thus, if it is determined that there is an undesired difference in the time of onset of myocardial relaxation between the sensed, different portions of the myocardium, i.e. the portions for which the sensors are sensitive for cardiac wall movements, then the timing parameters of the stimulation pulse delivery may be amended such that a synchronized onset of myocardial relaxation is provided.

Furthermore, according to further exemplifying embodiments, changes in the timing parameters of the stimulation pulse delivery can be performed in order to reduce or avoid the occurrences of post-systolic contractions, PSC, which generally are localized events. As mentioned above, the occurrences of PSC during the diastolic phase, whether localized or involving the entire myocardium, will undoubtedly have a detrimental effect on the coronary flow locally. It may also disturb coronary flow that occurs in coronary arteries located downstream of those affected coronary arteries that are located in the myocardial region that contracts as a result of the PSC. Thus, by amending the timing parameters, suitably in a manner that has been previously determined to have an inhibiting effect on the occurrences of PSC, for instance at the time of implantation of the cardiac stimulator, the occurrences of undesired and detrimental PSC can be reduced and possibly even avoided.

Moreover, according to still further exemplifying embodiments, the changes in the timing parameters of the stimulation pulse delivery can be performed in order to lengthen the duration of simultaneous myocardial relaxation between the different portions of the myocardium for which cardiac wall movements are sensed. In other words, timing parameters are adjusted for obtaining as long time duration of simultaneous diastole as possible. Thereby, as long time as possible for enabling coronary flow to occur is provided. The detection and determination of simultaneous myocardial relaxation is interrupted if a PSC should occur within the diastolic phase. Thus, the term "simultaneous myocardial relaxation" refers to the time period from the onset of diastole for all sensed portions of the heart, until the onset of a myocardial contraction at any of the sensed portions of the heart, whether the contraction is a regular systolic contraction or an irregular post-systolic contraction.

As understood by the skilled person, the above described three objectives, i.e. simultaneous onset of myocardial relaxation, reduction or possible minimization of PSC, and extension or possible maximization of simultaneous duration of myocardial relaxation, is preferably combined when possible. Also, there may be situations when one must be prioritized over the other. Such prioritizing may be the subject of physician settings, or be pre-programmed shipping settings.

According to further embodiments of the invention, said three objectives can be adapted to the current heart rate, either intrinsic or stimulation induced stimulation. For example, it may be the aim for the changes in the timing parameters of the stimulation pulse delivery to achieve simultaneous onset at a basic heart rate, while at a higher heart rate the aim may be to extend the time duration of simultaneous myocardial relaxation, or vice versa.

Furthermore, according to still further exemplifying embodiments, if an asynchrony is detected at a high heart rate, the control circuitry of the cardiac stimulator may be arranged to reduce the heart rate. This may lead to a reduction of the cardiac output, but may on the other hand increase coronary flow since the time duration of diastole increases as a result of increased interval between cardiac contractions. Thus, for heart stimulators in which the pacing therapy may be automatically adjusted by the heart stimulator in order to optimize or maximize cardiac output, a synchronized and elongated diastolic phase may be given priority over the optimization of cardiac output. For instance, in patients suffering from ischemic heart disease, it may be more important to ensure synchronized diastole and, thereby, adequate coronary flow at all times rather than maximized cardiac output.

Suitable adjustments for avoiding or eliminating undesired diastolic asynchrony and restoring a suitable level of diastolic synchrony could according to exemplifying embodiments be determined or set prior to or during implantation of the cardiac stimulator. In such embodiments, sensor signals could be registered during a pre-set stimulation protocol, and synchronization index then be calculated for different pre-set stimulation conditions and settings. Examples of such pre-set stimulation protocols triggering changes in the timing parameters of stimulation pulse delivery could include an AV stimulation protocol with varying AV delays, a VV stimulation protocol with varying VV delays, etc. During implantation, i.e. when the heart is studied with the sensors during no stimulation and various stimulation protocols, the expected changes in synchronization index at heart decompensation can be outlined. The term decompensation refers to a situation where changes in the demand for cardiac output of the patient, for instance when the patient rises from a sitting to a standing position or switches from walking to running, is not compensated for by the heart.

In further embodiments, the indication of diastolic asynchrony could be used for triggering an alarm signal to the patient. This alarm signal could be intended for prompting the patient to seek medical assistance for care or follow-up.

It should be understood that the indication of diastolic asynchrony does not have to be a binary value. On the contrary, the asynchrony indication preferably also provides information of the severity of asynchrony. Thus, the threshold value as referred to above, may in fact be a number of threshold values. For instance, a first value could be an indication of slight asynchrony to be used for diagnostic purposes, a second value could trigger a change in the pacing therapy, and a third value could be used for triggering an alarm to the patient that he needs to see his/her physician.

Furthermore, the change in timing parameters for the delivery of stimulation pulses may be performed in adaptation to the synchronization signal, or to the output signals from the wall movement sensors. Thus, the synchronization signal may provide an indication of the loss of synchrony, as well as further information which will provide information on how the lost synchrony is to be recovered. Furthermore, the sensor output signals may for instance provide information of the cause of the asynchrony. For instance, occurrences of diastolic asynchrony detected by two out of three sensors may provide an indication of where the source of the asynchrony occurred or originated. Moreover, the sensor output signals and/or the synchronization may suitably be stored for future use by the physician for diagnosis of occurred events at follow-up.

The monitoring of diastolic synchrony and/or detection of diastolic asynchrony is preferably performed at predetermined time intervals. As an example, the monitoring could be performed by receiving the output signals from the sensors, and providing a diastolic synchronization signal at any suitable predetermined or varied time interval given the current cardiac situation, e.g. once a week, every three days, once a day, every 8 hours, etc. Preferably, the time interval is set such that monitoring is performed often enough for an asynchrony to be detected at such an early stage that corrective action may be immediately taken and possible detrimental effects avoided, and seldom enough such that an unduly large energy consumption resulting from the monitoring procedure may be avoided.

In some embodiments, the time interval may be varied in adaptation to expected possible changes in the function of the heart. For instance, it may be expected that variations and changes in the myocardial function is most likely to occur, and to occur most frequently, in the time immediately following implantation. Thus, the monitoring interval can be set at a short interval for the period immediately following implant, and then be automatically extended as the time from implantation increases.

Furthermore, the time interval may be shortened as a result of detected changes in the diastolic synchrony. Thus, following a change in diastolic synchrony, possibly into asynchrony with ensuing corrective actions taking place, the time interval is suitably decreased in order to frequently monitor for possible further deterioration, or for an improvement as a result of the corrective actions taken.

Moreover, immediately following a change in the pacing therapy, determinations of possible asynchrony should take place. Also, if the synchronization signal or index is a quantitative value directly indicating the level of synchrony, the change in synchronization based on any pacing therapy variations can be monitored and the pacing therapy further adjusted accordingly.

In further examples, if the monitoring should indicate that diastolic asynchrony has arisen, then one or more further measurements and determinations of diastolic synchronization may be performed before an indication of diastolic asynchrony is provided and possible ensuing actions are initiated. Thereby, a sudden, isolated asynchronous event will not have an undue impact on the overall stimulation pacing therapy.

In exemplifying embodiments of the present invention, the synchronization monitoring is based on the output of two sensors, in which a first sensor is positioned at a location related to the right ventricle of the heart and arranged for sensing cardiac wall movements of the right ventricle, and the second sensor is positioned at a location related to the left ventricle of the heart and arranged for sensing cardiac wall movements of the left ventricle. Preferably, the right ventricle sensor is positioned within the right ventricle and attached to the ventricular wall, and the left ventricle sensor is positioned in the coronary sinus region outside the left ventricle and in contact with the left ventricular wall. In this example, interventricular diastolic synchronization is monitored. As used herein, the phrase "coronary sinus region" refers to the vasculature of the left ventricle, including any portion of the coronary sinus, great cardiac vein, left marginal vein, left lateral vein, left posterior ventricular vein, middle cardiac vein, and/or small cardiac vein or any other cardiac vein accessible via the coronary sinus.

In further exemplifying embodiments of the present invention, the synchronization monitoring is based on the output of two sensors positioned in or at the same ventricle. Then, the sensors are suitably used for monitoring intraventricular diastolic synchronization, for instance in the right ventricle. However, if one sensor is positioned within the right ventricle and attached to the cardiac septum, or in the immediate vicinity thereof, the sensor could be arranged to sense cardiac wall movements related to myocardial contraction and relaxation originating from the left ventricle.

Moreover, one or more additional sensors could in further examples of the invention be provided in or at the left or the right ventricle for providing additional output signal(s) on which the monitoring of diastolic synchronization is based. Also, sensors could be provided in the atrium for delivering output signal(s) on which the diastolic synchronization is based.

It should be noted that a number of different sensors could be used in the context of this invention for sensing cardiac wall movements, which are known per se to the person skilled in the art. For example, the sensors could be in the form of accelerometers, of any suitable type, or in the form of piezoelectric pressure transducers. Thus, the scope of the present invention is not restricted to the particular sensor alternatives disclosed herein.

In the examples that will be presented in the following, the sensors are provided at the distal end of cardiac leads that are arranged for providing stimulation pulses to the atria and/or ventricles of the heart, or for conducting sensed intrinsic cardiac signals from the heart to the heart stimulator. However, it should be noted that sensors provided on separate implantable leads, or other implantable devices, are also contemplated in the context of this application. Thus, the scope of the present invention is not restricted to sensors arranged on such implantable leads for stimulation and sensing as will be discussed below.

Further objects and advantages of the present invention will be discussed below by means of exemplifying embodiments.

### .Brief description of the drawings

Exemplifying embodiments of the invention will be described below with reference to the accompanying drawings, in which:
Fig. 1 is a simplified, partly cutaway view illustrating an implantable stimulator according to one exemplifying embodiment of the present invention;
Figs. 2 and 3 are partly cut-away views of a human heart provided with leads and sensors according to further exemplifying embodiments;
Fig. 4 is an illustration in a block diagram form of an implantable stimulator according to the embodiment shown in Fig. 1;
Fig. 5 is an illustration in a block diagram form of an analysis device arranged to receive signals from three cardiac wall motion sensors;
Figs. 6a-6c are schematic illustrations of the determination of diastolic synchrony according to embodiments of the present invention,
Figs. 7a-7c are schematic illustrations corresponding to those of Figs. 6a-6c, but in which a diastolic asynchrony is determined;
Figs. 8a-8c illustrate the determination of a diastolic asynchrony resulting from a local post-systolic contraction;
Figs. 9a-9d illustrate examples of sensor positions where all sensors are positioned at the left ventricle;
Figs. 10a-10d illustrate examples of sensor positions where sensors are positioned at both the right and the left ventricle, respectively;
Figs. 11a and 11b illustrate in diagram form a first example of how a synchronization index may be obtained; and
Figs. 12a and 12b illustrate in diagram form a second example of how a synchronization index may be obtained.

### Description of exemplifying embodiments

The following is a description of exemplifying embodiments in accordance with the present invention. This description is not to be taken in a limiting sense, but is made merely for the purpose of describing the general principles of the invention. Thus, even though particular types of heart stimulators will be described, such as biventricular pacemakers with or without atrial sensing and/or stimulation, the invention is also applicable to other types of cardiac stimulators, such as univentricular or dual chamber pacemakers, implantable cardioverter defibrillators (ICD's), etc.

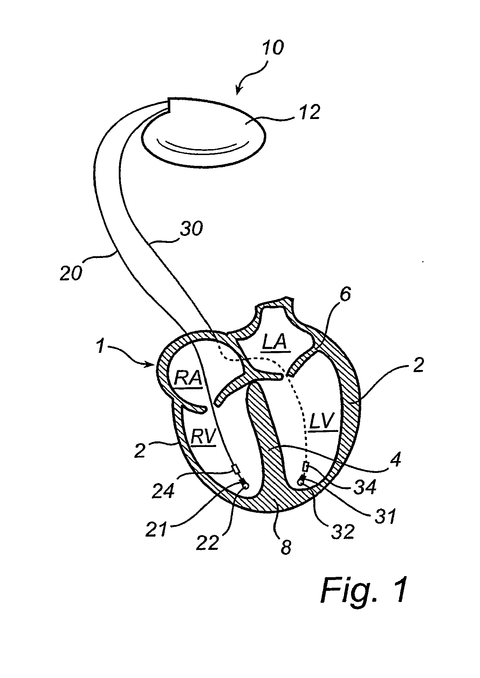

With reference first to fig. 1, there is shown a stimulation device 10 in electrical communication with a patient's heart 1 by way of two leads 20 and 30 suitable for delivering multi-chamber stimulation (and possible shock therapy). The heart illustrated portions of the heart 1 include the right atrium RA, the right ventricle RV, the left atrium LA, the left ventricle LV, cardiac walls 2, the ventricular septum 4, the valve plane 6, and the apex 8. The valve plane 6 refers to the annulus fibrosis plane separating the ventricles from the atria and containing all four heart valves, i.e. the aortic, pulmonary, mitral, and tricuspid valves.

In order to sense right ventricular cardiac signals and to provide stimulation therapy to the right ventricle RV, the stimulation device 10 is coupled to an implantable right ventricular lead 20 having a ventricular tip electrode 22, a ventricular annular or ring electrode 24, and a cardiac wall movement sensor 21. The ring electrode 24 is arranged for sensing electrical activity, intrinsic or evoked, in the right ventricle RV. The right ventricular tip electrode 22 is arranged to be implanted in the endocardium of the right ventricle, e.g. near the apex 8 of the heart. Thereby, the tip electrode 22 becomes attached to the cardiac wall and follows the cardiac wall movements, which movements can be sensed by the sensor 21 arranged near the tip electrode. In this example, the sensor is fixedly mounted in a distal header portion of the lead 20, in which the tip electrode 22 is also fixedly mounted. Furthermore in this example, the sensor is in the form of an accelerometer. However, other arrangements sensor types are contemplated for the cardiac wall motion sensor 21.

In order to sense left ventricular cardiac signals and to provide pacing therapy for the left ventricle LV, the stimulation device 10 is coupled to a "coronary sinus" lead 30 designed for placement via the coronary sinus in veins located distally thereof, so as to place a distal electrode adjacent to the left ventricle. Also, additional electrode(s) (not shown) could thereby be positioned adjacent to the left atrium. The coronary sinus lead 30 is designed to receive ventricular cardiac signals from the cardiac stimulator 10 and to deliver left ventricular LV pacing therapy using at least a left ventricular tip electrode 32 to the heart 1. In the illustrated example the LV lead 30 comprises an annular ring electrode 34 for sensing electrical activity related to the left ventricle LV of the heart. Furthermore, a cardiac wall movement sensor 31 is arranged at the tip electrode 32 for sensing left ventricular LV cardiac wall movements.

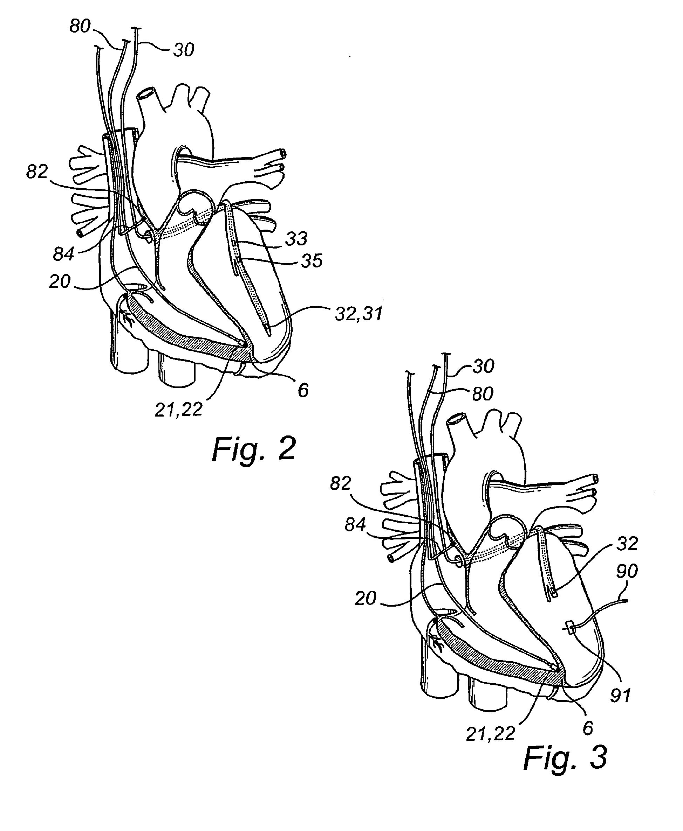

Turning briefly to Figs. 2 and 3, two alternative embodiments for placement of cardiac leads, cardiac electrodes and cardiac wall movement sensors for sensing longitudinal valve plane movements are illustrated. In Fig. 2, the RV and LV leads 20, 30 have been supplemented with a right atrial RA lead 80. The lead comprises an RA tip electrode 82 positioned in the patient's right atrial appendage for delivering electrical stimuli to the right atrium, and an RA ring electrode 84 for sensing and conducting cardiac signals from the right atrium to the cardiac stimulator. A cardiac wall motion sensor is provided at the RA tip electrode 82 for sensing cardiac wall movements of the RA wall. Furthermore, the LV lead 30 is provided with an additional cardiac wall movement sensor 33 arranged at the valve plane 6, as well as an additional stimulating electrode, of the ring type, .arranged distally of the movement sensor 33. Thereby, cardiac wall movements at a plurality of locations, i.e. three or four, may be sensed and conducted via the cardiac leads 20, 30, 80 to the cardiac stimulator.

Furthermore, Fig. 3 illustrates yet another example of lead, electrode and sensor placements. Here, the RV, RA and LV leads 20, 30 and 80 have been supplemented by an external epicardial lead 90 connected to the implantable stimulator 10. The epicardial lead 90 may be arranged for delivering stimulation pulses to the left ventricle LV of the heart, but is in this example only arranged for sensing cardiac wall movements and comprises a cardiac wall motion sensor 91. Thus, even though the LV lead 30 terminates and the stimulation electrode 32 for stimulation of the left ventricle arranged at a position near the valve plane 6 of the heart, local wall movements occurring in the LV cardiac wall further down towards the apex 8 may still be sensed.

Even though three examples have been illustrated in Figs. 1-3, the invention is not restricted to the illustrated examples of lead, electrode and sensor placement. For example, several epicardial electrodes and/or wall motion sensors could be used, wall motion sensors could be arranged at plural positions in the ventricles only, all wall motion sensors could be arranged in the same ventricle, plural atrial wall sensors could be used, etc. Also, in the illustrated examples, the wall motion sensors are of accelerometer type. However, other types of sensors for sensing and measuring wall movements are to be comprised in the scope of the present application. Further examples of sensor placements will be presented in relation to the further embodiments that will be described below.

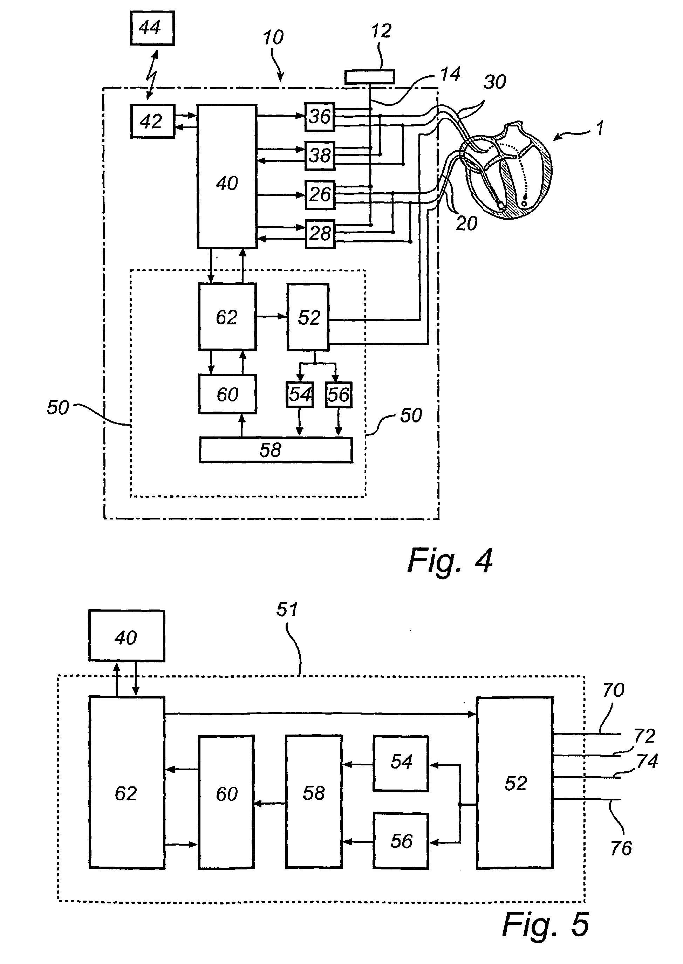

Turning now to Fig. 4, the heart stimulator 10 of Fig. 1 is shown in a block diagram form. For illustrative purposes, reference is made to Fig. 1 for the elements of the leads that are intended for positioning in or at the heart. The heart stimulator 10 is connected to a heart 1 in order to sense heart signals and emit stimulation pulses to the heart 1. A first tip electrode 22 is anchored in the right ventricle RV of the heart 1 and connected, via a first electrode conductor in the lead 20, to a first pulse generator 26 in the heart stimulator 2. A first ring electrode 24 is connected near the first tip electrode 22 and, via a second electrode conductor in the first lead 20, to the first pulse generator 26. A stimulation pulse to the right ventricle can be delivered to heart tissue by the first pulse generator via the first lead 20 and the first tip electrode 22. The stimulation pulse is then returned, via the first ring electrode 24 and the first lead 20, to the first pulse generator 26. Alternately, the stimulation pulse can be delivered via the first tip electrode 22 and an indifferent electrode 12 which, in this instance, consists of the enclosure of the heart stimulator 10 but can also consist of a separate electrode located somewhere in the body. The indifferent electrode 12 is connected to the first pulse generator 26 via an electrode conductor 14 in order to return stimulation pulses from the right ventricle. A first detector 28 is connected in parallel across the output terminal of the first pulse generator 26 in order to sense right ventricular activity in the heart.

In corresponding manner, a second tip electrode 32 is positioned in a vein distally of the coronary sinus and, thus, connected to the left ventricle LV of the heart 1, and, via a conductor in the second lead 30, to a second pulse generator 36. A second ring electrode 34 is located near the second tip electrode 32 and connected, via a further conductor in the second electrode lead 30, to the second pulse generator 36. Delivery of a stimulation pulse to the ventricle can be bipolar via the second tip electrode 32 and the second ring electrode 34, or unipolar via the second tip electrode 32 and the indifferent electrode 12. A second detector 38 is connected in parallel across the output terminal of the second pulse generator 36 in order to sense left ventricular activity in the heart. The pulse generators 26 and 36 and the detectors 28 and 38 are controlled by a control unit 40 which regulates the stimulation pulses with respect to amplitude, duration and stimulation interval, the sensitivity of the detectors 28 and 38 etc.

A physician using an extracorporeal programmer 56 can, via a telemetry unit 54, communicate with the heart stimulator 10 and thereby obtain information on identified conditions and also reprogram the different functions of the heart stimulator 10.

Fig. 4 further shows a first embodiment of the analysis device. The analysis device 50 is connected to the first sensor 21 via the first electrode lead 20, and to the second sensor via the second electrode lead 30. The analysis device 50 includes a measurement unit 52 which is capable of selectively receiving signals from any of the sensors, and which filters and amplifies the incoming signals in an appropriate manner, e.g. such that sensor signal contribution resulting from the systolic phase is removed from the measurement unit output signal.

The output signal from the measurement unit 52, which is proportional to the measurement signal, is then sent to a buffer 54 and to a differentiating circuit 56. Buffering is performed so that the differentiated signal is in phase with the proportional signal when they are sent to a calculator unit 58. The calculator unit 58 calculates a diastolic synchronization or synchrony value or signal based on the output signals from the respective sensors. The calculated synchronization signal 58 is sent to a comparator 60 for comparison with a threshold value, for instance indicative of when insufficient diastolic synchrony is present.

The output signal from the comparator comprises information of whether the synchronization signal passes the threshold value, or one of the threshold values for embodiments where a plurality of threshold values are utilized, and is forwarded to a microprocessor 62 which communicates with the control unit 40. If, e.g., an arisen asynchrony is identified, the control device 40 can institute therapeutic treatment with stimulation pulses in order to restore diastolic synchrony. The microprocessor 62 further controls the measurement unit 52 with respect to the measurement signal to be sent to the analysis device 50 and can also control the comparator 60, for example for varying threshold values in response to altered pacing therapy or due to altered settings by the physician.

With reference now to Fig. 5, there is shown an alternative analysis device 51. This alternative analysis device 51 basically comprises the same or similar elements as described in relation to the measurement unit analysis device 50 of Fig. 4. However, the alternative analysis device 51 is arranged for receiving output signals from three cardiac wall motion sensors via conductors 70, 72 and 74, the analysis device thus being arranged to provide a synchronization signal indicative of diastolic synchrony between three different locations of the heart.

Furthermore, a fourth conductor 76 provides an IEGM signal for the measurement unit. The IEGM signal is used by the analysis device 50, or rather by the differentiating circuit 54 and the calculator unit 58, as an aid in discriminating between the systolic and the diastolic phases of the heart cycle. Thereby, the analysis device can be arranged to only process sensor output signals provided during the diastolic phase. Thus, there will be no risk of misinterpreting an asynchrony that may be present in the systolic phase as an asynchrony in the diastolic phase.

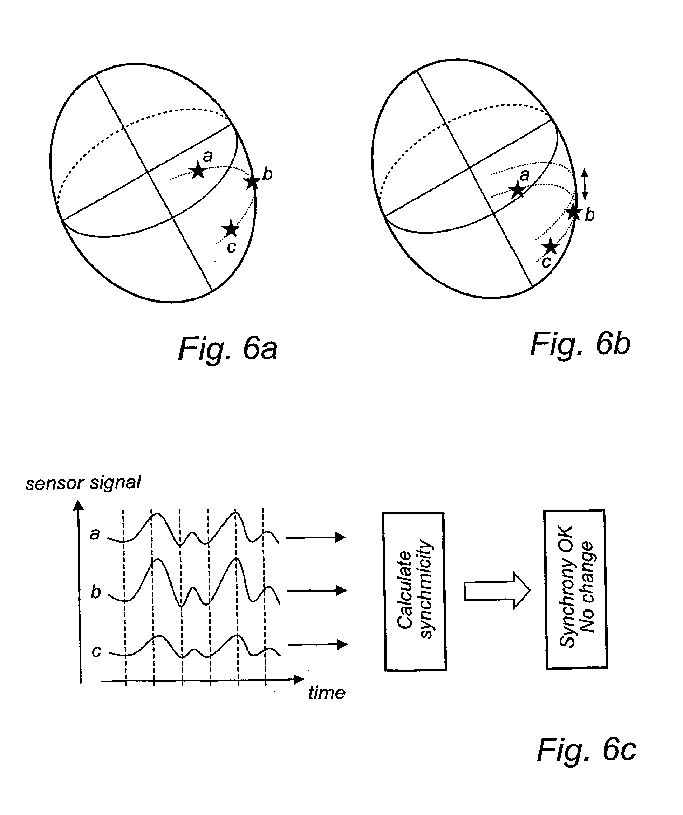

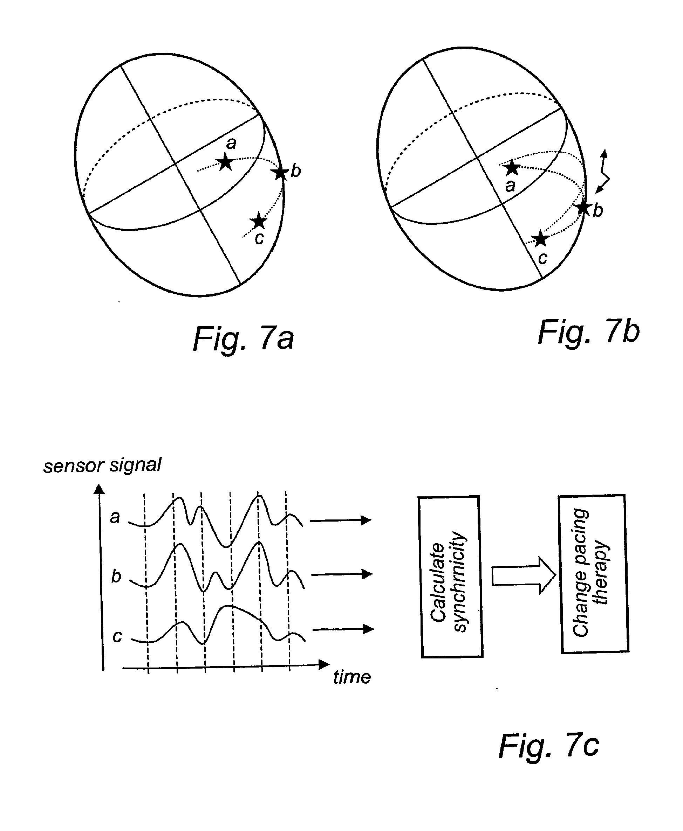

Turning now to Figs. 6-6c and 7a-7c, there will be shown in schematic form the presence and determination of diastolic synchrony and asynchrony, respectively. In Figs. 6a, 6b, 7a, and 7b, a heart is schematically illustrated with three cardiac wall motion sensors a, b and c positioned in the left ventricle LV of the heart.

In Fig. 6b, the position of the sensors, i.e. the cardiac wall portions in which the sensors are arranged, are illustrated at the instant of myocardial relaxation onset. Thus, even though myocardial relaxation has been initiated, the sensors and the wall portions thereof are still in a respective position obtained during myocardial contraction, i.e. immediately before movement of the cardiac wall tissue to the position during relaxation. In Fig. 6a, the very instant when the myocardium has just become fully dilated at the state of myocardial relaxation is illustrated. Thus, the movement of the sensors and the wall portions into the dilated positions have just ceased. The output signals of the sensors are illustrated in Fig. 6c, and it can be seen that the movements sensed by the three sensors are substantially simultaneous. Therefore, the processing circuitry, or analysis device, of the cardiac stimulator determines that there is diastolic synchrony. As a consequence, no further actions related to change in pacing therapy is performed.

In Fig. 7a, the position of the sensors and the respective cardiac wall portions thereof at instant of myocardial relaxation onset. Furthermore, at the instant depicted in Fig. 7b c, only sensors a and c have reached the position of the obtained in the fully dilated state of the myocardium. Sensor b, and the cardiac wall portion to which sensor b is attached, is on the other hand still in a position obtained during myocardial contraction. Hence, there is lack in synchrony between the cardiac wall portions at which the sensor a, b and c are attached, respectively. In particular, it seem to be a difference in the time of myocardial relaxation onset between the cardiac wall portion sensed by sensor b as compared to the cardiac wall portions sensed by sensors a and c. This lack in synchrony also appears in the output signals a, b and c of the cardiac wall motion sensors a, b and c, respectively. Thus, upon performing a synchronicity analysis for the output signals in the diastolic phase of the heart cycle, it can be determined that diastolic asynchrony is present and that suitable measures should be taken. Such measures could include restoring the cardiac synchrony or to derive an alarm signal indicative of the diastolic asynchrony.

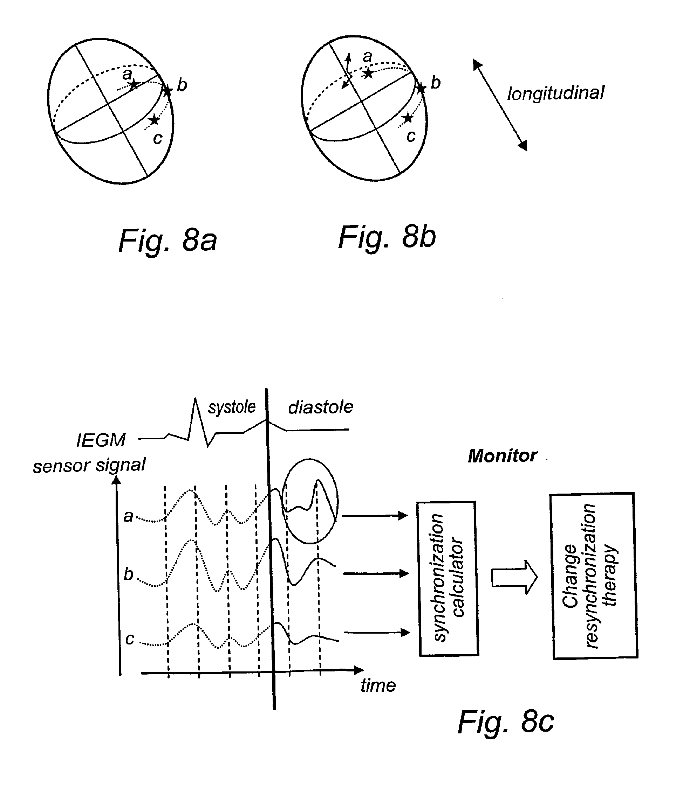

Turning now to Figs. 8a-8c, there is shown a further example of the occurrence and detection of diastolic asynchrony. In Fig. 8a, the positions of the cardiac wall motion sensors a, b and c at an instant where the myocardium has assumed a dilated state is shown. In Fig. 8b, a post-systolic contraction PSC occurs in the portion where sensor a is arranged for sensing cardiac wall movements. Consequently, sensor a is subjected to a movement at an instant when sensors b and c remain substantially stationary. This appears in the combined sensor signal outputs a, b and c and can be detected and determined as an asynchrony in the diastolic phase by the analysis device 50 of the stimulator 10. In the signal diagram of Fig. 8c, the portion comprising the signal output during the PSC is encircled. Thus, as a result of the determined asynchrony, appropriate adjustment of the pacing therapy may be executed in order to restore the diastolic synchrony.

In Figs. 6a through 8b, substantially only one example of the positioning of cardiac wall motion sensors is provided. However, there are a vast number of sensor positioning alternatives that are contemplated within the scope of the present application. In fact, any placement of sensors for measuring cardiac wall motions occurring during the diastolic phase of the heart cycle may be used, as long as there is in fact movements of the particular portion to which the sensor is located and attached during the diastolic phase. Thus, the present application are not limited to a particular number of wall motion sensors, or to particular positioning thereof.

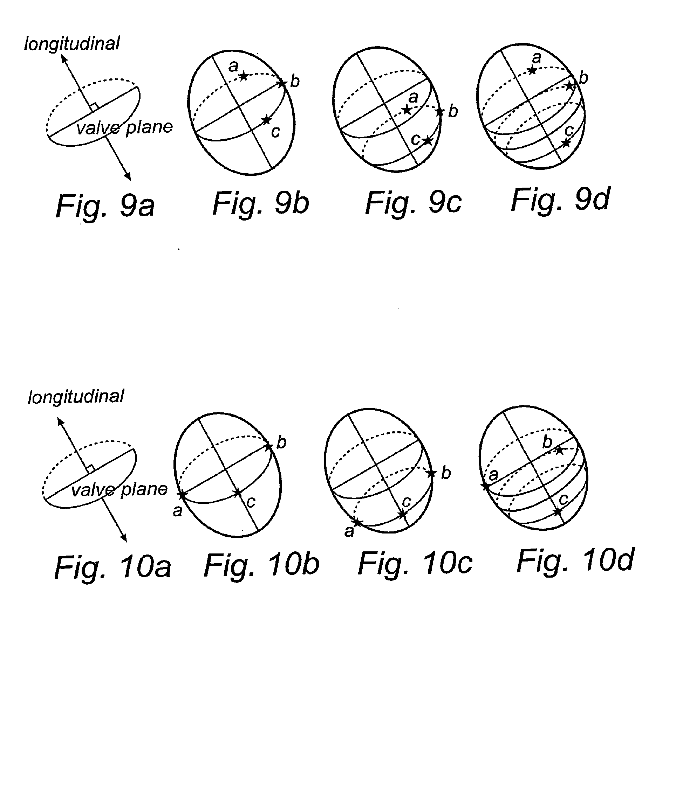

Turning to Figs. 9a-9d, further examples of wall motion sensors are provided. In these examples, the sensors a, b and c are arranged at the same ventricle, i.e. for measuring cardiac wall movements at several locations in the left ventricle LV of the heart. Fig. 9a is intended to illustrate the orientation of the valve plane, which is indicated by numeral 6 in Fig. 1. In the example illustrated in Fig. 9b, the sensors are positioned in the actual valve plane. Then, the sensors could in one alternative be positioned in the actual annulus fibrosis tissue, or epicardially outside the annulus fibrosis plane.

In Fig. 9c and 9d, two alternative examples of sensor positionings are presented. In Fig. 9c, the sensors a, b and c have been positioned at equal distances from the valve plane, thus forming a sensor plane parallel to the valve plane. Thereby, the sensors are assumed to be subjected to movements of substantially the same distance during the heart cycle, which may be beneficial when calculating and determining synchrony and possible sudden or expected appearance of asynchrony. In the example shown in Fig. 9d, the sensors are positioned at different levels at one ventricle along the longitudinal axis, or long-axis, of the heart. In this example, the physician has positioned the sensors at selected regions of interest, for instance regions suffering from a conductive disorder or having hibernating tissue which is expected or suspected to become active during remodulation of the heart due to progressing stimulation therapy.

Turning now to Figs. 10a to 10d, further examples of sensor positioning are illustrated. In the examples, the sensors are arranged in or at both ventricles of the heart. First, Fig. 10a illustrates the valve plane and the longitudinal direction of the heart. Then, Fig. 10b illustrates the example where the cardiac wall motion sensors are positioned and arranged to sense cardiac wall movements at the valve plane. In the same manner as mentioned above in relation to Fig. 9b, the sensors could in one alternative be positioned in the actual annulus fibrosis tissue, or epicardially outside the annulus fibrosis plane. Suitable, the right ventricular sensor a is arranged endocardially in the valve plane, and the left ventricular sensor c is arranged epicardially. The sensor b arranged at the septum 4 could be arranged epicardially either directly or via a coronary vein, or endocardially, via the right atrium and ventricle. Possibly, the RV sensor a arranged at the valve plane could be replaced for an RA sensor arranged in or at the valve plane, e.g. in the annulus fibrosis tissue.

In fig. 10c, the sensors a, b and c have been positioned in or at the right and the left ventricle, respectively, at equal distances from the valve plane, thus forming a sensor plane parallel to the valve plane. Thereby, similar to the example shown in Fig. 9c, the sensors are assumed to be subjected to movements of substantially the same distance during the heart cycle, which may be beneficial when calculating and determining synchrony and possible sudden or expected appearance of asynchrony.

Finally, in the example illustrated in Fig. 10d, the sensors are positioned at different levels, in or at the right and the left ventricle, along the longitudinal axis of the heart. In this example, similar to the example shown in Fig. 9d, the physician has positioned the sensors at selected regions of interest, for instance regions suffering from a conductive disorder or having hibernating tissue which is expected or suspected to become active during remodulation of the heart due to progressing stimulation therapy.

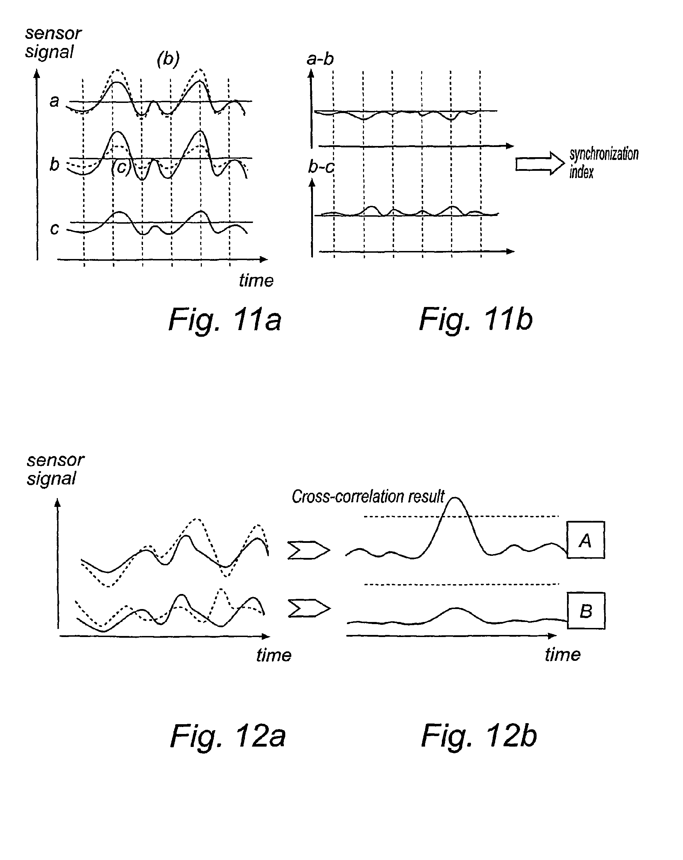

When the signal output from the sensors a, b and c is received by the analysis device 50, a calculation of a synchronization index or signal is performed, which can be used for determining synchrony of the heart. In Fig. 11a, the output signals a, b and c, stemming from the sensors a, b and c, respectively, indicative of cardiac wall movements are illustrated in a diagram. In the portion of the diagram illustrating sensor output signal a, the sensor output signal b has been added as shown by the dotted line. Similarly, the sensor output signal c has been added to the portion of the diagram illustrating sensor output signal b. In this example, the difference between the sensor output signals a and b and the difference between the sensor output signals is calculated for the diastolic phase. This is performed by simply subtracting sensor output signal b from a and sensor output signal c from b.

The resulting difference signals are shown in Fig. 11b. These signals could be further added to each other in order to arrive at the synchronization index or signal. Alternatively, the difference signals could be used separately in order to provide dual synchronization indices or signals. Furthermore, statistical calculations could be applied to the difference signal(s) to arrive at a suitable value indicative of the synchronization.

Fig. 12a and 12b illustrate a further example of deriving one or more synchronization indices or signals. Here, the upper and lower portions of the diagram in Fig. 12a illustrates two signals obtained from two sensor output signals, respectively. One signal is indicated with a solid line, and the other one with a dotted line, respectively. These pairs of sensor output signals are cross-correlated in order to arrive at a cross-correlation result which is used as said synchronization indices or signals. In the illustrated example, two cross-correlation results in the form of synchronization index A and synchronization index B are obtained. The synchronization signals can then be compared with a threshold value, which is illustrated in Fig. 12b with the dotted straight line, and appropriate measures be taken when the synchronization signal exceeds the threshold level.

When the monitoring of diastolic synchronization has revealed that a diastolic asynchrony has arisen, or that a reduction of diastolic synchrony has occurred, the parameters for timing of stimulation pulse delivery may be changed in order to restore or improve the diastolic synchrony. Such a change could for example refer to an adjustment in the VV-interval, e.g. for a biventricular heart stimulator; a change in the AV-interval, e.g. for a dual chamber or an AV-sequential heart stimulator; or combinations thereof. Thereby, the diastolic synchrony can be monitored and the pacing therapy adjusted such that the coronary flow may be maintained at a desired level.

In further embodiments, the indication of diastolic asynchrony could be used for triggering an alarm signal to the patient. This alarm signal could be intended for prompting the patient to seek medical assistance for care or follow-up.

It should be noted that the sensors may be subjected to pressures, movements and/or accelerations that are not derived from or related to the intrinsic movements of the myocardium and the cardiac walls thereof. For instance, accelerations derived from extra-cardiac movements of the patient, such as from running, vibrations in the patient environment, thoracic movements etc. However, output signal contributions deriving from intrinsic movements of the myocardial tissue can easily be discriminated from signal contributions from such extra-cardiac movements since the latter have a substantially identical impact on the respective sensor. Furthermore, by designing the sensors to be sensitive for certain frequency ranges, the majority of the extra-cardiac signal contributions may be omitted. Furthermore, band-pass filtering of the sensor outputs may also be used for discriminating or filter out the signal contribution from extra-cardiac movements.

While the invention disclosed herein has been described by means of specific embodiments and applications thereof, numerous modifications and variations could be made therein by those skilled in the art without departing from the scope of the invention, which is defined by the appended claims.

## Claims

1. A device (50) for monitoring the heart cycle of a human heart (1) such that coronary flow may be maintained at a desired level, said device (50) being connected to a first sensor (21) adapted to be positioned at a first location of said human heart (1) and arranged for sensing cardiac wall movements at said first location, and to a second sensor (31) adapted to be positioned at a second location of said human heart (1) and arranged for sensing cardiac wall movements at said second location, comprising:
processing circuitry (52, 54, 56, 58, 60) arranged for receiving output signals from said first sensor (21) and said second sensors (31), said output signals being indicative of myocardial relaxation at said first and second locations; **characterized in that**
said processing circuitry (52, 54, 56, 58, 60) is arranged for determining the time of myocardial relaxation at said first and second locations; and
said processing circuitry (52, 54, 56, 58, 60) is arranged for providing a diastolic synchronization signal indicative of the synchrony in said time of myocardial relaxation between said first location and said second location.

2. The device as claimed in claim 1, wherein said processing circuitry (52, 54, 56, 58, 60) is arranged for determining said time of myocardial relaxation as the time when myocardial relaxation initially occurs after myocardial contraction.

3. The device as claimed in claim 1 or 2, wherein said processing circuitry (52, 54, 56, 58, 60) is arranged for determining said time of myocardial relaxation as the time duration of myocardial relaxation that is uninterrupted by myocardial contraction.

4. The device as claimed in claim 3, wherein
said processing circuitry (52, 54, 56, 58, 60) is arranged for determining said time duration of simultaneous myocardial relaxation at said first and second locations, and
said processing circuitry (52, 54, 56, 58, 60) is arranged for providing said diastolic synchronization signal holding information of said determined simultaneous myocardial relaxation.

5. The device as claimed in any one of the preceding claims, wherein
said device (50) is connectable to a third sensor adapted to be positioned at a third location of said human heart (1) and arranged for sensing cardiac wall movements at said third location, and
said processing circuitry (52, 54, 56, 58, 60) is further arranged for receiving output signals from said third sensor, said output signals being indicative of myocardial relaxation at said third location,
said processing circuitry (52, 54, 56, 58, 60) is arranged for determining the time of myocardial relaxation at said third location, and
said processing circuitry (52, 54, 56, 58, 60) is arranged for providing said diastolic synchronization signal indicative of the synchrony in said time of myocardial relaxation between said first, second and third locations.

6. The device as claimed in any one of the preceding claims, wherein said first sensor (21) and said second sensors (31) are positioned for sensing cardiac wall movements of a first ventricle of said human heart (1).

7. The device as claimed in any one of claims 1-5, wherein
said first sensor (21) is positioned for sensing cardiac wall movements of a first ventricle of said human heart (1), and
said second sensor (31) is positioned for sensing cardiac wall movements of a second ventricle of said human heart (1).

8. An implantable cardiac stimulator (10) for delivering stimulation pulses to a human heart (1), comprising:
a housing;
a pulse generator (26) enclosed in said housing for generating said stimulation pulses;
control circuitry (40) for controlling the delivery of said stimulation pulses to said human heart (1);
said implantable cardiac stimulator (10) being connected to a lead arrangement (20, 30, 80) for conducting said stimulation pulses to said human heart (1), and for conducting sensed electrical signals from said human heart (1) to said control circuitry (40); and
a device (50) for monitoring the heart cycle of a human heart as claimed in any one of claims 1-7.

9. The implantable cardiac stimulator as claimed in claim 8, wherein
said implantable cardiac stimulator (10) is arranged for receiving parameters for timing of stimulation pulse delivery for providing coronary flow at a desired level at implantation,
said implantable cardiac stimulator (10) is arranged for monitoring said heart cycle for determining the synchrony in the time of myocardial relaxation after implantation, and
said implantable cardiac stimulator (10) is arranged for adapting said parameters on the basis of said monitoring for maintaining said coronary flow at said desired level.

10. The implantable cardiac stimulator as claimed in claim 9, wherein said control circuitry (40) is arranged for adjusting the timing of stimulation pulse delivery when said processing circuitry (52, 54, 56, 58, 60) indicates that sufficient diastolic synchrony is no longer present.

11. The implantable cardiac stimulator as claimed in claim 9 or 10, wherein said control circuitry (40) is arranged for adjusting said timing in adaptation to said diastolic synchronization signal.

12. The implantable cardiac stimulator as claimed in any one of claims 9-10, wherein
said device (50) for monitoring said heart cycle is arranged for measuring the diastolic synchrony, and
said device (50) for monitoring said heart cycle is arranged for providing an output of whether sufficient diastolic synchrony is present after an adjustment of said timing of stimulation pulse delivery.

13. The implantable cardiac stimulator as claimed in any one of claims 9-12, wherein said implantable cardiac stimulator (10) is arranged for delivering stimulation pulses to both ventricles of said human heart (1), and wherein said timing of stimulation pulse delivery includes the V-V interval.

14. The implantable cardiac stimulator as claimed in any one of claims 9-13, wherein said implantable cardiac stimulator (10) is arranged for delivering stimulation pulses to a ventricle of said human heart (1), and for delivering stimulation pulses and/or sensing cardiac events in an atrium of said human heart (1), and wherein said timing of stimulation pulse delivery includes the A-V interval.

## Patentansprüche

1. Vorrichtung (50) zur Überwachung des Herzzyklus eines menschlichen Herzens (1), sodass die Koronardurchblutung auf einem gewünschten Niveau gehalten werden kann, wobei die Vorrichtung (50) mit einem ersten Sensor (21) verbunden ist, der so ausgelegt ist, dass er an einer ersten Stelle des menschlichen Herzens (1) platziert wird und so angeordnet wird, dass er Herzwandbewegungen an der ersten Stelle erfasst, und mit einem zweiten Sensor (31), der so ausgelegt ist, dass er an einer zweiten Stelle des menschlichen Herzens (1) platziert wird und so angeordnet wird, dass er Herzwandbewegungen an der zweiten Stelle erfasst, umfassend:
Verarbeitungsschaltkreise (52, 54, 56, 58, 60), die so angeordnet sind, dass sie Ausgangssignale von dem ersten Sensor (21) und dem zweiten Sensor (31) empfangen, wobei die Ausgangssignale eine Herzmuskelerschlaffung an der ersten und zweiten Stelle anzeigen; **dadurch gekennzeichnet, dass**
die Verarbeitungsschaltkreise (52, 54, 56, 58, 60) so angeordnet sind, dass sie den Zeitpunkt der Herzmuskelerschlaffung an der ersten und zweiten Stelle bestimmen; und
die Verarbeitungsschaltkreise (52, 54, 56, 58, 60) so angeordnet sind, dass sie ein Diastolensynchronisierungssignal liefern, das die Synchronität des Zeitpunkts der Herzmuskelerschlaffung zwischen der ersten Stelle und der zweiten Stelle liefern.

2. Vorrichtung nach Anspruch 1, wobei die Verarbeitungsschaltkreise (52, 54, 56, 58, 60) so angeordnet sind, dass sie den Zeitpunkt der Herzmuskelerschlaffung als den Zeitpunkt bestimmen, wenn es erstmals nach der Herzmuskelkontraktion zur Herzmuskelerschlaffung kommt.

3. Vorrichtung nach Anspruch 1 oder 2, wobei die Verarbeitungsschaltkreise (52, 54, 56, 58, 60) so angeordnet sind, dass sie den Zeitpunkt der Herzmuskelerschlaffung als die Zeitdauer der Herzmuskelerschlaffung bestimmen, die nicht durch eine Herzmuskelkontraktion unterbrochen wird.

4. Vorrichtung nach Anspruch 3, wobei
die Verarbeitungsschaltkreise (52, 54, 56, 58, 60) so angeordnet sind, dass sie die Zeitdauer der gleichzeitigen Herzmuskelerschlaffung an der ersten und zweiten Stelle bestimmen und
die Verarbeitungsschaltkreise (52, 54, 56, 58, 60) so angeordnet sind, dass sie das Diastolensynchronisierungssignal liefern, das Informationen über die bestimmte gleichzeitige Herzmuskelerschlaffung enthält.

5. Vorrichtung nach einem der vorhergehenden Ansprüche, wobei
die Vorrichtung (50) mit einem dritten Sensor verbindbar ist, der so ausgelegt ist, dass er an einer dritten Stelle des menschlichen Herzens (1) platziert wird und so angeordnet wird, dass er Herzwandbewegungen an der dritten Stelle erfasst, und
die Verarbeitungsschaltkreise (52, 54, 56, 58, 60) ferner so angeordnet sind, dass sie Ausgangssignale von dem dritten Sensor empfangen, wobei die Ausgangssignale eine Herzmuskelerschlaffung an der dritten Stelle anzeigen,
die Verarbeitungsschaltkreise (52, 54, 56, 58, 60) so angeordnet sind, dass sie den Zeitpunkt der Herzmuskelerschlaffung an der dritten Stelle bestimmen, und
die Verarbeitungsschaltkreise (52, 54, 56, 58, 60) so angeordnet sind, dass sie das Diastolensynchronisierungssignal liefern, das die Synchronität des Zeitpunkts der Herzmuskelerschlaffung zwischen der ersten, zweiten und dritten Stelle anzeigt.

6. Vorrichtung nach einem der vorhergehenden Ansprüche, wobei der erste Sensor (21) und der zweite Sensor (31) so platziert sind, dass sie Herzwandbewegungen einer ersten Herzkammer des menschlichen Herzens (1) erfassen.

7. Vorrichtung nach einem der Ansprüche 1 bis 5, wobei
der erste Sensor (21) so platziert ist, dass er Herzwandbewegungen einer ersten Herzkammer des menschlichen Herzens (1) erfasst, und
der zweite Sensor (31) so platziert ist, dass er Herzwandbewegungen einer zweiten Herzkammer des menschlichen Herzens (1) erfasst.

8. Implantierbarer Herzstimulator (10) zum Abgeben von Stimulationsimpulsen an ein menschliches Herz (1), umfassend:
ein Gehäuse;
ein Impulsgenerator (26) zum Erzeugen der Stimulationsimpulse, der in dem Gehäuse eingeschlossen ist;
Steuerschaltkreise (40) zum Steuern der Abgabe der Stimulationsimpulse an das menschliche Herz (1);
wobei der implantierbare Herzstimulator (10) an eine Leitungsanordnung (20, 30, 80) zum Leiten der Stimulationsimpulse zu dem menschlichen Herzen (1) und zum Leiten erfasster elektrischer Signale von dem Herzen (1) an die Steuerschaltkreise (40) angeschlossen ist; und
eine Vorrichtung (50) zum Überwachen des Herzzyklus eines menschlichen Herzens nach einem der Ansprüche 1 bis 7.

9. Implantierbarer Herzstimulator nach Anspruch 8, wobei
der implantierbare Herzstimulator (10) so angeordnet ist, dass er Parameter für die Festlegung des Zeitpunkts der Abgabe von Stimulationsimpulsen für die Bereitstellung der Koronardurchblutung auf einem gewünschten Niveau bei der Implantation empfängt,
der implantierbare Herzstimulator (10) so angeordnet ist, dass er den Herzzyklus überwacht, damit er die Synchronität des Zeitpunkts der Herzmuskelerschlaffung nach der Implantation bestimmt, und
der implantierbare Herzstimulator (10) so angeordnet ist, dass er die Parameter auf der Grundlage der Überwachung anpasst, damit die Koronardurchblutung auf dem gewünschten Niveau beibehalten wird.

10. Implantierbarer Herzstimulator nach Anspruch 9, wobei die Steuerschaltkreise (40) so angeordnet sind, dass sie den Zeitpunkt der Abgabe von Stimulationsimpulsen anpassen, wenn die Verarbeitungsschaltkreise (52, 54, 56, 58, 60) angeben, dass keine ausreichende Diastolensynchronität mehr vorhanden ist.

11. Implantierbarer Herzstimulator nach Anspruch 9 oder 10, wobei die Steuerschaltkreise (40) so angeordnet sind, dass sie den Zeitpunkt angepasst an das Diastolensynchronisierungssignal anpassen.

12. Implantierbarer Herzstimulator nach Anspruch 9 oder 10, wobei
die Vorrichtung (50) zum Überwachen des Herzzyklus zum Messen der Diastolensynchronität angeordnet ist, und
die Vorrichtung (50) zum Überwachen des Herzzyklus so angeordnet ist, dass sie ausgibt, ob eine ausreichende Diastolensynchronität nach einer Anpassung des Zeitpunkts der Abgabe von Stimulationsimpulsen vorhanden ist.

13. Implantierbarer Herzstimulator nach einem der Ansprüche 9 bis 12, wobei der implantierbare Herzstimulator (10) so angeordnet ist, dass er an beide Herzkammern des menschlichen Herzens (1) Stimulationsimpulse abgibt, und wobei der Zeitpunkt der Abgabe von Stimulationsimpulsen das VV-Intervall enthält.

14. Implantierbarer Herzstimulator nach einem der Ansprüche 9 bis 13, wobei der implantierbare Herzstimulator (10) so angeordnet ist, dass er Stimulationsimpulse an eine Herzkammer des menschlichen Herzens (1) abgibt und dass er in einem Vorhof des menschlichen Herzens (1) Stimulationsimpulse abgibt und/oder Herzereignisse erfasst, und wobei der Zeitpunkt der Abgabe von Stimulationsimpulsen das AV-Intervall enthält.

## Revendications

1. Dispositif (50) de surveillance du cycle cardiaque d'un coeur humain (1) de telle manière que le débit coronaire puisse être maintenu à un niveau souhaité, ledit dispositif (50) étant connecté à un premier capteur (21) adapté pour être positionné à un premier emplacement dudit coeur humain (1) et agencé pour détecter des mouvements de la paroi cardiaque audit premier emplacement, et à un deuxième capteur (31) adapté pour être positionné à un deuxième emplacement dudit coeur humain (1) et agencé pour détecter des mouvements de la paroi cardiaque audit deuxième emplacement, comprenant :
des circuits de traitement (52, 54, 56, 58, 60) agencés pour recevoir des signaux de sortie dudit premier capteur (21) et dudit deuxième capteur (31), lesdits signaux de sortie indiquant une relaxation myocardique auxdits premier et deuxième emplacements ; **caractérisé en ce que**
lesdits circuits de traitement (52, 54, 56, 58, 60) sont agencés pour déterminer le temps de relaxation myocardique auxdits premier et deuxième emplacements ; et
lesdits circuits de traitement (52, 54, 56, 58, 60) sont agencés pour fournir un signal de synchronisation diastolique indiquant la synchronicité dudit temps de relaxation myocardique entre ledit premier emplacement et ledit deuxième emplacement.

2. Dispositif selon la revendication 1, dans lequel lesdits circuits de traitement (52, 54, 56, 58, 60) sont agencés pour déterminer ledit temps de relaxation myocardique en tant que le moment où la relaxation myocardique se produit initialement après la contraction myocardique.

3. Dispositif selon la revendication 1 ou 2, dans lequel lesdits circuits de traitement (52, 54, 56, 58, 60) sont agencés pour déterminer ledit temps de relaxation myocardique en tant que la durée de la relaxation myocardique qui n'est pas interrompue par la contraction myocardique.

4. Dispositif selon la revendication 3, dans lequel
lesdits circuits de traitement (52, 54, 56, 58, 60) sont agencés pour déterminer ladite durée de relaxation myocardique simultanée auxdits premier et deuxième emplacements, et
lesdits circuits de traitement (52, 54, 56, 58, 60) sont agencés pour fournir ledit signal de synchronisation diastolique contenant des informations sur ladite relaxation myocardique simultanée déterminée.

5. Dispositif selon l'une quelconque des revendications précédentes, dans lequel
ledit dispositif (50) peut être connecté à un troisième capteur adapté pour être positionné à un troisième emplacement dudit coeur humain (1) et agencé pour détecter des mouvements de la paroi cardiaque audit troisième emplacement, et
lesdits circuits de traitement (52, 54, 56, 58, 60) sont en outre agencés pour recevoir des signaux de sortie dudit troisième capteur, lesdits signaux de sortie indiquant une relaxation myocardique audit troisième emplacement,
lesdits circuits de traitement (52, 54, 56, 58, 60) sont agencés pour déterminer le temps de relaxation myocardique audit troisième emplacement, et
lesdits circuits de traitement (52, 54, 56, 58, 60) sont agencés pour fournir ledit signal de synchronisation diastolique indiquant la synchronicité dudit temps de relaxation myocardique entre lesdits premier, deuxième et troisième emplacements.

6. Dispositif selon l'une quelconque des revendications précédentes, dans lequel ledit premier capteur (21) et ledit deuxième capteur (31) sont positionnés pour détecter des mouvements de la paroi cardiaque d'un premier ventricule dudit coeur humain (1).

7. Dispositif selon l'une quelconque des revendications 1 à 5, dans lequel
ledit premier capteur (21) est positionné pour détecter des mouvements de la paroi cardiaque d'un premier ventricule dudit coeur humain (1), et
ledit deuxième capteur (31) est positionné pour détecter des mouvements de la paroi cardiaque d'un second ventricule dudit coeur humain (1).

8. Stimulateur cardiaque implantable (10) destiné à transmettre des impulsions de stimulation à un coeur humain (1), comprenant :
un boîtier ;
un générateur d'impulsions (26) enfermé dans ledit boîtier pour générer lesdites impulsions de stimulation ;
des circuits de commande (40) destinés à commander la transmission desdites impulsions de stimulation audit coeur humain (1) ;
ledit stimulateur cardiaque implantable (10) étant connecté à un agencement de dérivations (20, 30, 80) destiné à conduire lesdites impulsions de stimulation audit coeur humain (1), et à conduire des signaux électriques détectés dudit coeur humain (1) auxdits circuits de commande (40) ; et
un dispositif (50) de surveillance du cycle cardiaque d'un coeur humain selon l'une quelconque des revendications 1 à 7.

9. Stimulateur cardiaque implantable selon la revendication 8, dans lequel
ledit stimulateur cardiaque implantable (10) est agencé pour recevoir des paramètres pour établir le moment de transmission d'impulsions de stimulation afin d'assurer un débit coronaire à un niveau souhaité à l'implantation,
ledit stimulateur cardiaque implantable (10) est agencé pour surveiller ledit cycle cardiaque afin de déterminer la synchronicité du temps de relaxation myocardique après l'implantation, et
ledit stimulateur cardiaque implantable (10) est agencé pour adapter lesdits paramètres sur la base de ladite surveillance pour maintenir ledit débit coronaire audit niveau souhaité.

10. Stimulateur cardiaque implantable selon la revendication 9, dans lequel lesdits circuits de commande (40) sont agencés pour ajuster le moment de transmission d'impulsions de stimulation quand lesdits circuits de traitement (52, 54, 56, 58, 60) indiquent qu'une synchronicité diastolique suffisante n'est plus présente.

11. Stimulateur cardiaque implantable selon la revendication 9 ou 10, dans lequel lesdits circuits de commande (40) sont agencés pour ajuster ledit moment en fonction dudit signal de synchronisation diastolique.

12. Stimulateur cardiaque implantable selon l'une quelconque des revendications 9-10, dans lequel
ledit dispositif (50) de surveillance dudit cycle cardiaque est agencé pour mesurer la synchronicité diastolique, et
ledit dispositif (50) de surveillance dudit cycle cardiaque est agencé pour fournir une sortie indiquant si une synchronicité diastolique suffisante est présente après un ajustement dudit moment de transmission d'impulsions de stimulation.

13. Stimulateur cardiaque implantable selon l'une quelconque des revendications 9 à 12, dans lequel ledit stimulateur cardiaque implantable (10) est agencé pour transmettre des impulsions de stimulation aux deux ventricules dudit coeur humain (1), et dans lequel ledit moment de transmission d'impulsions de stimulation inclut l'intervalle V-V.

14. Stimulateur cardiaque implantable selon l'une quelconque des revendications 9 à 13, dans lequel ledit stimulateur cardiaque implantable (10) est agencé pour transmettre des impulsions de stimulation à un ventricule dudit coeur humain (1), et pour transmettre des impulsions de stimulation et/ou détecter des événements cardiaques dans une oreillette dudit coeur humain (1), et dans lequel ledit moment de transmission d'impulsions de stimulation inclut l'intervalle A-V.
